# FASCICULE DE BREVET EUROPEEN

(11) **EP 2 347 790 B1**
(45) Date de publication et mention de la délivrance du brevet: **02.01.2013**
(21) Numéro de dépôt: 10192746.5
(22) Date de dépôt: 26.11.2010
(51) Int. Cl.: A61N 1/37, A61N 1/08

(54) **Prothèse cardiaque implantable comprenant des moyens de détection et de protection contre les champs magnétiques forts produits par les imageurs IRM**
Implantierbare Herzprothese, die mit Erkennungs- und Schutzmitteln gegen starke Magnetfelder von Kernspintomographen ausgestattet ist
Implantable prosthetic heart including a means for detecting and protecting against the strong magnetic fields generated by MRI machines

(30) Priorité: 20.01.2010 FR 1050374
(43) Date de publication de la demande: 27.07.2011
(73) Titulaire: Sorin CRM SAS, 92143 Clamart Cedex (FR)
(72) Inventeur: Legay, Thierry, 91640, FONTENAY LES BRIIS (FR); Decoene, Dominique, 78760 Jouars Pontchartrain (FR)
(74) Mandataire: Dupuis-Latour, Dominique

(56) Documents cités:
- EP-A1- 1 935 450
- WO-A1-2006/081434
- WO-A2-2006/124481
- US-A- 6 101 417
- US-A1- 2006 167 496
- US-A1- 2009 138 058

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Ces dispositifs comportent un boîtier généralement désigné "générateur", raccordé électriquement et mécaniquement à une ou plusieurs "sondes" munie(s) d'électrodes destinées à venir en contact avec le myocarde sur des sites de recueil des potentiels électriques et/ou d'application des impulsions de stimulation.

L'invention concerne plus particulièrement les techniques permettant de sécuriser ces dispositifs lorsque le porteur doit être soumis à un examen par imagerie par résonance magnétique (IRM ou MRI).

En effet, un examen IRM est aujourd'hui en principe contre-indiqué aux patients porteurs d'un stimulateur ou défibrillateur cardiaque (ci-après "dispositif"), et ce pour plusieurs raisons :
- le risque d'échauffement à proximité des électrodes reliant le générateur au coeur du patient ;
- les forces et couples d'attraction exercés sur le dispositif plongé dans le champ magnétique statique très élevé de l'imageur IRM ;
- un comportement imprédictible du dispositif lui-même, du fait de l'exposition à ces champs magnétiques extrêmes.

La présente invention a pour objet d'apporter une solution au troisième type de problème.

Il est en effet souhaitable que lors de l'exposition au champ électromagnétique (statique et alternatif) généré par l'imageur IRM, le dispositif ait un comportement documenté et connu au préalable.

En effet, en l'absence de mesure particulière, le dispositif peut être sujet à divers phénomènes, notamment :
- une détection erratique du champ statique fort généré par l'imageur IRM, en particulier pour les dispositifs pourvus d'un détecteur de champ magnétique de type interrupteur à lame souple (ILS). Un tel interrupteur, conçu pour détecter des champs magnétiques statiques relativement faibles (présence d'un aimant permanent placé à proximité de l'implant par un praticien pour mettre temporairement le dispositif dans un mode de fonctionnement spécifique), présente un comportement totalement imprévisible dans un environnement d'imagerie IRM, où les champs magnétiques sont souvent plus de mille fois supérieurs à ceux d'un aimant permanent ;
- une dégradation de ses performances intrinsèques ;
- une inhibition de la fonction de stimulation par des signaux dynamiques émis par l'imageur IRM détectés par le dispositif et interprétés à tort comme des signaux cardiaques ;
- une altération des impulsions de stimulation envoyées par le dispositif et/ou la présence de stimuli parasites.

Pendant toute la durée de l'examen - qui peut se prolonger plusieurs minutes -, le dispositif doit néanmoins rester fonctionnel et assurer si nécessaire et sans discontinuité la stimulation du myocarde. Il est donc indispensable de disposer de moyens de détection et de gestion d'une telle situation, assurant les fonctions suivantes :
- indication au dispositif que le patient va être soumis à un examen IRM ;
- inhibition des circuits du dispositif susceptibles d'être perturbés par les champs électromagnétiques émis par l'imageur IRM ;
- fonctionnement dans un mode de stimulation dédié, adapté au patient et compatible avec les champs électromagnétiques produits par l'imageur.

Des techniques particulières ont été proposées pour détecter automatiquement des champs magnétiques statiques forts tels que ceux produits par les imageurs IRM, avec un ordre de grandeur du tesla (typiquement entre 0,5 T et 3 T, voire plus). Les détecteurs de champ faible dont sont équipées la plupart des dispositifs, sensibles à la présence d'un aimant (champ statique typiquement de l'ordre de 1,5 mT), sont en effet inadaptés à la détection des champs critiques forts tels que ceux produits par les imageurs IRM qui sont donc près de mille fois plus élevés, et se situent dans des zones de réponse non linéaire de ces capteurs, qui risquent donc d'être "sourds" à la présence d'un champ fort.

Le US 2007/191914 A1 décrit un dispositif dans lequel la présence d'un champ magnétique statique fort est détectée par analyse de l'impédance d'un composant inductif, par exemple de l'une des bobines d'un régulateur inductif à découpage : la présence d'un champ magnétique fort a pour effet de saturer le noyau de ce composant, provoquant un changement d'impédance qui peut être signalé au dispositif.

Le WO 2006/124481 A2 décrit une autre technique, consistant à détecter la présence d'un champ IRM par mesure de la tension recueillie aux bornes d'une antenne de télémétrie ainsi que sur la sonde.

Le EP 1 935 450 A1 décrit une autre technique encore, consistant à utiliser des capteurs de type résistance magnétique géante (RMG ou GMR) associés en un pont de Wheatstone. Ce pont joue un rôle de capteur unique mixte champ fort/champ faible : en effet, l'équilibre du pont se trouve plus ou moins altéré selon l'amplitude du champ, et les modifications résultantes de la tension différentielle peuvent être analysées par un convertisseur placé en sortie du pont pour donner une estimation globale du niveau de champ.

Le US2009/0138058 A1 prévoit par ailleurs la possibilité de programmer le dispositif pour le placer dans un état d'attente d'examen IRM désigné *MRI Mode,* par exemple à l'occasion d'une consultation avec le cardiologue. Dans cet état d'attente, le fonctionnement du dispositif est un fonctionnement standard mais avec activation de la possibilité de détecter un champ magnétique fort. De la sorte, en présence d'un tel champ magnétique fort (c'est-à-dire au début de l'examen IRM), le dispositif basculera vers un mode protégé désigné *MRI-Safe Mode,* compatible avec le champ très élevé que subira le dispositif pendant la durée de l'examen IRM. Une fois l'examen achevé, le dispositif est reprogrammé vers le mode normal de fonctionnement, désigné *Normal Operation Mode.*

Ces diverses techniques impliquent l'ajout d'un capteur supplémentaire, ce qui nécessite de revoir la conception matérielle du dispositif, entraîne un surcoût, et crée une emprise supplémentaire sur les circuits, à l'encontre des exigences de miniaturisation extrême de ceux-ci.

L'un des buts de l'invention est de remédier à ces inconvénients, en proposant une nouvelle technique applicable notamment à des appareils conventionnels ou d'ancienne génération, ne possédant pas de capteur dédié à la détection d'un champ statique fort tel que celui généré par un imageur IRM.

On verra en particulier que la technique de l'invention peut être mise en oeuvre par une simple adaptation logicielle du microcontrôleur d'un dispositif existant, donc sans qu'il soit nécessaire de modifier la conception matérielle, et de ce fait procure une très grande économie de moyens, sans surcoût notable.

Le principe de base de l'invention consiste à utiliser le capteur de champ magnétique déjà présent dans le dispositif et destiné à détecter un champ magnétique statique faible (typiquement de l'ordre du millitesla) correspondant à la détection d'un aimant permanent approché de l'implant en vue de placer celui-ci dans un "mode à l'aimant" spécifique.

Ce capteur sera utilisé sans modification matérielle pour, selon l'invention, placer le dispositif dans un état d'attente d'examen IRM (ci-après "état d'attente"), par exemple à l'occasion d'une consultation cardiologique.

En effet, si le patient doit subir un examen IRM, un rendez-vous lui sera donné par le centre de radiologie. Préalablement à ce rendez-vous, le patient ira consulter son cardiologue, qui placera le dispositif en état d'attente d'examen IRM.

Dans cet état d'attente d'examen IRM, le fonctionnement du dispositif est initialement un fonctionnement standard, jusqu'à ce que se produise l'un des deux événements suivants :
- lorsque le patient entre dans la salle où est situé l'imageur IRM, le dispositif détecte un champ statique relativement élevé : bien que notablement inférieur au champ qui sera généré par l'imageur dans le corps du patient au moment de l'examen proprement dit, un tel champ est toujours présent dans la salle même quand aucun examen n'est en cours ; et/ou
- quelques instants avant l'entrée du patient dans la salle abritant l'imageur IRM, un aimant permanent est appliqué par un opérateur sur le thorax du patient dans la région du dispositif.

Cet événement aura pour effet de placer le dispositif dans un mode de fonctionnement spécifique, modifié par rapport au fonctionnement standard et compatible avec le champ très élevé que subira dispositif lorsque le patient passera l'examen IRM.

On notera que le passage dans le mode de fonctionnement spécifique est opéré juste avant le début de l'examen IRM proprement dit, donc de manière préventive, automatiquement au moment de l'entrée dans la salle abritant l'appareil IRM ou sur commande spécifique (par application d'un aimant).

Une fois l'examen achevé et le niveau de champ magnétique ambiant retombé à une valeur résiduelle très faible, le dispositif retournera automatiquement à son mode de fonctionnement normal, sans qu'il soit en aucune façon nécessaire d'intervenir spécifiquement à cet effet, par exemple par une reprogrammation du dispositif comme pour le US2009/0138058 A1 précité.

Avantageusement, lorsque le dispositif est basculé vers l'état d'attente IRM, le cardiologue programme une temporisation avec une durée qu'il peut choisir (en pratique, de quelques heures à quelques semaines). La temporisation est activée dès ce moment, le dispositif décomptant alors automatiquement la durée programmée. Au terme de cette durée, par sécurité le dispositif quittera automatiquement l'état d'attente sans que le patient n'ait besoin de consulter à nouveau son cardiologue.

Plus précisément, l'invention propose un dispositif du type général divulgué par le US2009/0138058 A1 précité, comprenant les éléments énoncés au préambule de la revendication 1.

L'invention propose, pour remédier aux problèmes exposés plus haut, les caractéristiques énoncées à la partie caractérisante de la revendication 1.

Les sous-revendications visent des modes de réalisation subsidiaires avantageux.

On va maintenant décrire un exemple de mise en oeuvre de l'invention, en référence aux dessins annexés.
La Figure 1 est une vue schématique d'un générateur de dispositif implantable, montrant les éléments essentiels de ce dispositif.
La Figure 2 est un synoptique du fonctionnement du dispositif selon l'invention.

L'invention peut être mise en oeuvre par une modification simple du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés. L'adaptation de ces appareils à la mise en oeuvre des fonctions de l'invention est à la portée de l'homme du métier, et elle ne sera pas décrite en détail.

L'invention peut notamment être appliquée aux dispositifs implantables tels que ceux des familles *Reply* et *Paradym* produits et commercialisés par Sorin CRM, Clamart, France. Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous.

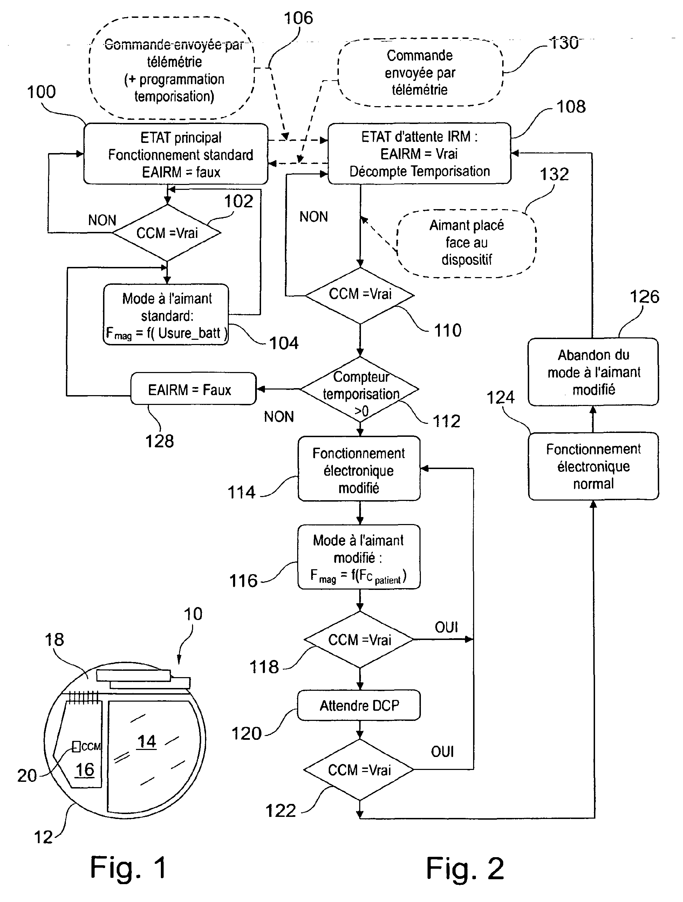

Sur la Figure 1, la référence 10 désigne de façon générale le générateur d'un dispositif implantable de type stimulateur cardiaque.
Cet implant comprend un boîtier 12 logeant une pile d'alimentation 14 et un circuit électronique 16 relié à un connecteur 18 sur lequel pourront être branchées une ou plusieurs sondes pourvues, à leur autre extrémité, d'électrodes de détection/stimulation.

Le dispositif est en outre pourvu d'un capteur de champ magnétique 20 destinés à détecter un champ magnétique statique faible (typiquement un champ de l'ordre du millitesla).

Sa technologie est l'une de celles couramment utilisées pour la détection d'un aimant permanent approché de l'implant en vue de placer celui-ci dans un "mode à l'aimant" spécifique. Le capteur 20 pourra être choisi parmi les composants de type : bobine dont le noyau sature en présence d'un champ magnétique faible, résistance magnétique géante (RMG), capteur à effet Hall, capteur intégré tel que composant de type MAGFET (comme décrit par exemple dans le WO 94/12238 A1), ou encore capteur de type microsystème électromécanique MEMS (comme décrit par exemple dans le FR 2 805 999 A1). On évitera les capteurs de type ILS comprenant des éléments métalliques, dont le comportement est imprédictible en présence de champs forts.

Ce capteur 20 est un capteur qui est déjà présent dans le dispositif et pourra être utilisé sans modification matérielle pour la mise en oeuvre de l'invention. Il délivre en sortie un signal binaire CCM qui sera 'vrai' ou 'faux' selon que le champ détecté est présent ou non, avec un seuil typiquement supérieur ou égal à 1 mT.

La Figure 2 est un organigramme exposant de façon détaillée la mise en oeuvre de la technique de l'invention.

Dans l'état initial (bloc 100) le dispositif est en mode de fonctionnement standard, non modifié. Un indicateur binaire EAIRM ({État d'Attente d'examen IRM) est positionné à 'faux'.

Si, dans cet état, le capteur 20 détecte un champ magnétique (CCM = 'vrai' au test 102), alors le dispositif passe en "mode à l'aimant" (bloc 104). Ce mode en lui-même bien connu, ci-après dénommé "mode à l'aimant standard" consiste essentiellement à stimuler le patient en mode DOO avec une fréquence de stimulation dépendant du niveau d'usure de la pile (ce qui permet de tester ce niveau).

Dès que l'aimant est retiré (CCM = 'faux' au test 102), le dispositif retourne dans son mode de fonctionnement principal standard programmé.

Ce fonctionnement est celui de l'état de l'art.

De façon caractéristique de l'invention, lorsqu'il est dans son état principal (bloc 100) le dispositif peut recevoir une commande 106 envoyée par télémétrie (télémétrie inductive ou télémétrie RF) sur ordre d'un cardiologue, qui le fera passer dans un état dit "état d'attente d'examen IRM" (bloc 108).

Cet état, ci-après "état d'attente" sera utilisé si le patient doit subir un examen IRM dans un laps de temps proche, compris typiquement entre quelques minutes et quelques semaines. L'indicateur EAIRM est positionné à 'vrai', et une durée de temporisation est programmée par le cardiologue, correspondant à la durée maximale pendant laquelle le dispositif sera autorisé à rester dans l'état d'attente. Le décompte de cette temporisation commence immédiatement dès le passage à l'état d'attente (ou, en variante, il peut être différé d'une durée également programmable).

Dans l'état d'attente, le mode de fonctionnement du dispositif est à ce stade un mode standard avec activation de toutes les fonctionnalités habituelles, à savoir le même mode de fonctionnement que dans l'état principal du bloc 100.

Ce mode standard est maintenu tant que le capteur de champ magnétique 20 ne détecte aucun champ statique (test 110, CCM = 'faux'). Si, en revanche, (i) le capteur détecte la présence d'un aimant (test 110, CCM = 'vrai') et (ii) la temporisation n'est pas échue (test 112), alors le dispositif considère que cette condition est un signal indiquant que le patient va être soumis à bref délai à un champ IRM fort.

Le dispositif va alors modifier son comportement électronique (bloc 114), notamment en désactivant un certain nombre de circuits utilisant directement ou indirectement des propriétés magnétiques, en particulier les circuits de télémétrie RF et les alimentations à découpage. Les systèmes d'alimentation seront alors basés sur des régulateurs linéaires de tension ou des convertisseurs capacitifs, consommant certes plus d'énergie mais insensibles aux effets des champs magnétiques.

Le mode de fonctionnement du dispositif sera également modifié (bloc 116), le dispositif passant dans un mode dit "mode à l'aimant modifié" dans lequel, notamment :
- la fréquence de stimulation n'est plus indexée sur l'usure de la pile (comme dans le "mode à l'aimant standard") mais par exemple sur la moyenne du rythme cardiaque du patient ;
- la fonction de détection est inhibée, pour éviter que les signaux dynamiques émis par l'imageur IRM ne soient interprétés à tort par le dispositif comme des signaux cardiaques ;
- dans le cas d'un défibrillateur implantable, toute délivrance de choc est inhibée.

Ce "mode à l'aimant modifié" est maintenu tant que dure l'examen IRM.
Le test de détection de champ magnétique de l'aimant est réitéré (test 118) et, si ce test est positif (CCM = 'vrai'), alors le système maintient le mode à l'aimant modifié. Dans le cas contraire (CCM = 'faux') le dispositif se met en attente de l'expiration d'un délai de confirmation programmable (DCP), par exemple de 10 minutes (bloc 120), délai à l'issue duquel le test de présence du champ magnétique est réitéré (test 122). Si ce test est positif (CCM = 'vrai'), le dispositif reste dans le mode à l'aimant modifié en cours ; dans le cas contraire (CCM = 'faux'), le fonctionnement électronique standard est rétabli (bloc 124, correspondant au rétablissement des fonctions modifiées au bloc 114) et le mode à l'aimant modifié est abandonné (bloc 126, correspondant au rétablissement des fonctions modifiées au bloc 116). La situation est donc celle qui prévalait avant détection d'un champ magnétique au test 110, à savoir un retour à l'état d'attente avec fonctionnement standard du bloc 108.

Si, lorsque le dispositif est en état d'attente (bloc (108), (i) le capteur détecte la présence d'un champ magnétique (test 110, CCM = 'vrai') mais (ii) que la temporisation est alors échue (test 112), alors cet état d'attente est abandonné (bloc 128, indicateur EAIRM positionné à 'faux'). Le dispositif revient au mode de fonctionnement de type "mode à l'aimant standard" (bloc 104), le test de présence du champ est réitéré et si ce test est négatif (test 102, CCM = 'faux'), le dispositif retourne à son état principal correspondant à la situation initiale (bloc 100), avant l'activation de l'état d'attente par le cardiologue.

Une autre manière de forcer le retour à cet état principal initial consiste à envoyer par télémétrie une commande 130 avant que la temporisation ne soit échue. Dans ce cas le temporisateur est désactivé et n'est pas pris en compte.

On notera que lorsque le dispositif est en état d'attente (bloc 108), le passage au mode de fonctionnement modifié sécurisé peut résulter non seulement de la détection automatique d'un champ magnétique ambiant relativement élevé régnant dans la salle abritant l'imageur IRM, comme on l'a décrit plus haut, mais peut être également forcé par un opérateur, au moyen d'un geste 132 consistant simplement à appliquer un aimant permanent sur le thorax du patient dans la région du dispositif.

Cette manière de procéder est particulièrement conseillée avec les imageurs IRM utilisés pour scanner seulement une partie du corps (les membres, la tête, ...), car dans ces situations le patient porte un vêtement blindé protégeant le tronc des champs dynamiques forts, ce qui pourrait empêcher une détection automatique du champ statique fort de l'imageur IRM. Le praticien a alors la possibilité de forcer le passage dans le fonctionnement électronique modifié et le mode à l'aimant modifié en forçant le test 110, en appliquant l'aimant permanent avant que le patient ne revête le vêtement de protection.

## Revendications

1. Un dispositif médical actif implantable (10) du type prothèse cardiaque de stimulation, resynchronisation et/ou défibrillation, comprenant :
- un circuit électronique de détection/stimulation (16) ;
- un capteur de champ magnétique (20) ;
- des moyens de mise en sécurité, aptes à faire basculer le dispositif :
. d'un mode standard (100) où les fonctions nominales du dispositif sont actives,
. vers un mode IRM protégé spécifique (114, 116) adapté à un fonctionnement en présence d'un champ magnétique statique d'un niveau correspondant à celui susceptible d'être émis par un imageur IRM,
le dispositif étant apte à retourner du mode IRM protégé spécifique au mode standard sur absence de détection (118, 122, 110) de champ magnétique par ledit capteur ;
- des moyens émetteurs/récepteurs de télémétrie, aptes à assurer un couplage du dispositif avec un appareil programmateur externe ; et
- des moyens (106) pour placer temporairement le dispositif, lorsque celui-ci est en mode standard (100), dans un état d'attente d'examen IRM (108),
dispositif **caractérisé en ce que** :
- ledit capteur de champ magnétique est un capteur de champ faible (20) apte à détecter (102, 110, 118, 122) la présence d'un champ magnétique statique d'un niveau correspondant à celui susceptible d'être émis par un aimant permanent approché (132) au voisinage du dispositif, et
- le dispositif est apte à passer :
• lorsqu'il est dans ledit mode standard (100), vers un mode à l'aimant (104) où la fréquence de stimulation dépend du niveau d'usure de la pile d'alimentation du dispositif,
• lorsqu'il est dans ledit état d'attente d'examen IRM (108), vers ledit mode IRM protégé spécifique (114, 116),
sur détection d'un champ magnétique par ledit capteur de champ faible (20).

2. Le dispositif de la revendication 1, dans lequel lesdits moyens de mise en sécurité sont activables via les moyens émetteurs/récepteurs de télémétrie (106) pour placer le dispositif en état d'attente d'examen iRM (108) lorsqu'il est dans le mode standard (100).

3. Le dispositif de la revendication 1 , comprenant_en_ outre :
- des moyens temporisateurs pour décompter un laps de temps prédéterminé à partir du placement du dispositif dans l'état d'attente d'examen IRM (108), et
- des moyens pour replacer automatiquement le dispositif dans le mode standard à l'expiration (112) du laps de temps décompté par les moyens temporisateurs.

4. Le dispositif de la revendication 1, comprenant en outre :
- des moyens (130) pour replacer le dispositif, lorsque celui-ci est en état d'attente d'examen IRM (108), dans le mode standard (100), ces moyens pour replacer le dispositif dans le mode standard étant des moyens activables via les moyens émetteurs/récepteurs de télémétrie. 3

5. Le dispositif de la revendication 3, dans lequel le laps de temps prédéterminé des moyens temporisateurs est une donnée programmable par ledit appareil programmateur externe et transmise (106) au dispositif via les moyens émetteurs/récepteurs de télémétrie.

6. Le dispositif de la revendication 1, comprenant en outre des moyens d'inhibition (120) du retour du dispositif depuis le mode IRM protégé spécifique vers le mode standard, ces moyens d'inhibition opérant pendant une durée prédéterminée (DCP) décomptée à partir du basculement du dispositif dans le mode IRM protégé spécifique (114, 116).

7. Le dispositif de la revendication 6, dans lequel ladite durée prédéterminée des moyens d'inhibition est une durée programmable par ledit appareil programmateur externe et transmise au dispositif via les moyens émetteurs/récepteurs de télémétrie.

8. Le dispositif de la revendication 1, dans lequel le capteur de champ faible (20) est un capteur apte à détecter la présence d'un champ magnétique statique d'un niveau d'au moins 1 mT.

9. dispositif de la revendication 1,_dans lequel le capteur de champ faible (20) est un capteur choisi dans le groupe formé par : bobine dont le noyau tend à saturer en présence d'un champ magnétique faible, résistance magnétique géante RMG, capteur à effet Hall, capteur MAGFET intégré, capteur MEMS.

10. Le dispositif de la revendication 1, dans lequel les moyens de mise en sécurité sont des moyens aptes, lorsqu'ils sont activés, à :
- mettre le circuit dans un mode de fonctionnement protégé standard de type mode à l'aimant sans détection ;
- inhiber les organes du dispositif sensibles aux effets délétères d'une exposition aux champs magnétiques statique et radiofréquence émis par un imageur IRM ; et
- produire des impulsions de stimulation à une fréquence prédéterminée, indépendante du niveau de charge de la pile d'alimentation du dispositif.

## Claims

1. Active implantable medical device (10) of the cardiac prosthesis type for stimulation, resynchronization and/or defibrillation, comprising:
- a detection/stimulation electronic circuit (16);
- a magnetic field sensor (20);
- protection means, suitable for switching the device:
• from a standard mode (100) in which the nominal functions of the device are active,
• to a specific protected MRI mode (114, 116) suitable for operation in the presence of a static magnetic field of a level corresponding to that likely to be emitted by an MRI imager,
the device being suitable for returning from the specific protected MRI mode to the standard mode on the absence of detection (118, 122, 110) of magnetic field by said sensor;
- telemetry transceiver means, suitable for ensuring a coupling of the device with an external programming apparatus; and
- means (106) for temporarily placing the device, when the latter is in standard mode (100), in a state awaiting MRI examination (108),
which device is **characterized in that**:
- said magnetic field sensor is a weak field sensor (20) suitable for detecting (102, 110, 118, 122) the presence of a static magnetic field of a level corresponding to that likely to be emitted by a nearby permanent magnet (132) in the vicinity of the device, and
- the device is suitable for switching:
• when it is in said standard mode (100), to a magnet mode (104) in which the stimulation frequency depends on the level of consumption of the power supply battery of the device,
• when it is in said state awaiting MRI examination (108), to said specific protected MRI mode (114, 116),
on detection of a magnetic field by said weak field sensor (20).

2. Device of Claim 1, in which said protection means can be activated via the telemetry transceiver means (106) to place the device in the state awaiting MRI examination (108) when it is in the standard mode (100).

3. Device of Claim 1, also comprising:
- timer means for counting down a predetermined time period from the placement of the device in the state awaiting MRI examination (108), and
- means for automatically replacing the device in the standard mode on expiry (112) of the time period counted down by the timer means.

4. Device of Claim 1, also comprising:
- means (130) for replacing the device, when the latter is in the state awaiting MRI examination (108), in the standard mode (100),
these means for replacing the device in the standard mode being means that can be activated via the telemetry transceiver means.

5. Device of Claim 3, in which the predetermined time period of the timer means is a datum that can be programmed by said external programmer apparatus and transmitted (106) to the device via the telemetry transceiver means.

6. Device of Claim 1, also comprising means (120) for inhibiting the return of the device from the specific protected MRI mode to the standard mode, these inhibition means operating for a predetermined duration (DCP) counted down from the switchover of the device to the specific protected MRI mode (114, 116).

7. Device of Claim 6, in which said predetermined duration of the inhibition means is a duration that can be programmed by said external programmer apparatus and transmitted to the device via the telemetry transceiver means.

8. Device of Claim 1, in which the weak field sensor (20) is a sensor suitable for detecting the presence of a static magnetic field of a level of at least 1 mT.

9. Device of Claim 1, in which the weak field sensor (20) is a sensor chosen from the group formed by: coil of which the core tends to saturate in the presence of a weak magnetic field, giant magnetoresistance GMR, Hall effect sensor, integrated MAGFET sensor, MEMS sensor.

10. Device of Claim 1, in which the protection means are means suitable, when they are activated, for:
- placing the circuit in a standard protected operating mode of the magnet mode without detection type;
- inhibiting the members of the device that are sensitive to the damaging effects of exposure to the static and radio frequency magnetic fields emitted by an MRI imager; and
- producing stimulation pulses at a predetermined frequency, independent of the level of charge of the power supply battery of the device.

## Patentansprüche

1. Implantierbare aktive medizinische Vorrichtung (10) des Typs Stimulations-, Resynchronisations- und/oder Defibrillations-Herzprothese, die Folgendes umfasst:
- eine elektronische Detektions-/Stimulationsschaltung (16);
- einen Magnetfeldsensor (20);
- Sicherungsmittel, die die Vorrichtung umschalten können von:
- einer Standardbetriebsart (100), in der die normalen Funktionen der Vorrichtung aktiv sind,
- zu einer spezifischen geschützten IRM-Betriebsart (114, 116), die für einen Betrieb in Gegenwart eines statischen Magnetfeldes mit einem Betrag, der jenem entspricht, der von einem IRM-Bildgeber emittiert werden kann, ausgelegt ist,
wobei die Vorrichtung dafür ausgelegt ist, bei Fehlen einer Detektion (118, 122, 110) des Magnetfeldes durch den Sensor von der spezifischen geschützten IRM-Betriebsart zu der Standardbetriebsart zurückzukehren;
- Telemetrie-Sender/Empfänger-Mittel, die dafür ausgelegt sind, eine Kopplung der Vorrichtung mit einer externen Programmiervorrichtung zu gewährleisten; und
- Mittel (106), um die Vorrichtung dann, wenn sie in der Standardbetriebsart (100) ist, vorübergehend in einen IRM-Untersuchungswartezustand (108) zu versetzen,
wobei die Vorrichtung **dadurch gekennzeichnet ist, dass**:
- der Magnetfeldsensor ein Sensor (20) für schwaches Feld ist, der das Vorhandensein eines statischen Magnetfelds mit einem Betrag, der jenem entspricht, der von einem in die Umgebung der Vorrichtung gebrachten Permanentmagneten (132) emittiert werden kann, detektieren (102, 110, 118, 122) kann, und
- die Vorrichtung dafür ausgelegt ist,
- dann, wenn sie in der Standardbetriebsart (100) ist, in eine Magnetbetriebsart (104) überzugehen, in der die Stimulationsfrequenz von dem Nutzungspegel der Versorgungsbatterie der Vorrichtung abhängt,
- dann, wenn sie in dem IRM-Untersuchungswartezustand (108) ist, in die spezifische geschützte IRM-Betriebsart (114, 116) überzugehen,
wenn ein Magnetfeld durch den Sensor (20) für schwaches Feld detektiert wird.

2. Vorrichtung nach Anspruch 1, wobei die Sicherungsmittel über die Telemetrie-Sender/Empfänger-Mittel (106) aktivierbar sind, um die Vorrichtung in den IRM-Untersuchungswartezustand (108) zu versetzen, wenn sie in der Standardbetriebsart (100) ist.

3. Vorrichtung nach Anspruch 1, die außerdem Folgendes umfasst:
- Verzögerungsmittel, um ein vorgegebenes Zeitintervall ausgehend von dem Versetzen der Vorrichtung in den IRM-Untersuchungswartezustand (108) herunterzuzählen, und
- Mittel, um die Vorrichtung bei Ablauf (112) des durch die Verzögerungsmittel heruntergezählten Zeitintervalls automatisch in die Standardbetriebsart zurückzubringen.

4. Vorrichtung nach Anspruch 1, die außerdem Folgendes umfasst:
- Mittel (130), um die Vorrichtung dann, wenn sie in dem IRM-Untersuchungswartezustand (108) ist, in die Standardbetriebsart (100) zurückzubringen,
wobei diese Mittel zum Zurückbringen der Vorrichtung in die Standardbetriebsart Mittel sind, die über die Telemetrie-Sender/Empfänger-Mittel aktivierbar sind.

5. Vorrichtung nach Anspruch 3, wobei das vorgegebene Zeitintervall der Verzögerungsmittel Daten sind, die durch die externe Programmiervorrichtung programmierbar sind und an die Vorrichtung über die Telemetrie-Sender/Empfänger-Mittel übertragen (106) werden können.

6. Vorrichtung nach Anspruch 1, die außerdem Mittel (120) zum Sperren der Rückkehr der Vorrichtung von der spezifischen geschützten IRM-Betriebsart in die Standardbetriebsart umfasst, wobei die Sperrmittel während einer vorgegebenen Dauer (DCP) arbeiten, die ausgehend vom Schalten der Vorrichtung in die spezifische geschützte IRM-Betriebsart (114, 116) heruntergezählt wird.

7. Vorrichtung nach Anspruch 6, wobei die vorgegebene Dauer der Sperrmittel eine Dauer ist, die durch die externe Programmiervorrichtung programmierbar ist und an die Vorrichtung über die Telemetrie-Sender/Empfänger-Mittel übertragen werden kann.

8. Vorrichtung nach Anspruch 1, wobei der Sensor (20) für schwaches Feld ein Sensor ist, der das Vorhandensein eines statischen Magnetfeldes mit einem Betrag von wenigstens 1 mT detektieren kann.

9. Vorrichtung nach Anspruch 1, wobei der Sensor (20) für schwaches Feld ein Sensor ist, der aus der Gruppe gewählt ist, die gebildet ist aus: Spule, deren Kern in Gegenwart eines schwachen Magnetfeldes zu einer Sättigung neigt, magnetischer Riesenwiderstand (RMG), Hall-Effekt-Sensor, integrierter MAGFET-Sensor, MEMS-Sensor.

10. Vorrichtung nach Anspruch 1, wobei die Sicherungsmittel Mittel sind, die dann, wenn sie aktiviert sind, in der Lage sind:
- die Schaltung in eine geschützte Standardbetriebsart des Typs Magnetbetriebsart ohne Detektion zu versetzen;
- die Organe der Vorrichtung, die für schädliche Auswirkungen der Beaufschlagung mit statischen und hochfrequenten Magnetfeldern, die von einem IRM-Bildgeber emittiert werden, empfindlich sind, zu sperren; und
- Stimulationsimpulse mit einer vorgegebenen Frequenz zu erzeugen, die unabhängig von der Ladung der Versorgungsbatterie der Vorrichtung ist.
